# EUROPEAN PATENT SPECIFICATION

(11) **EP 3 740 140 B1**
(45) Date of publication and mention of the grant of the patent: **18.02.2026**
(21) Application number: 17933184.8
(22) Date of filing: 09.11.2017
(51) Int. Cl.: A61B 17/128

(54) **CLIP PUSHING MECHANISM FOR SURGICAL CLIP APPLICATOR**
KLAMMERSCHIEBEMECHANISMUS FÜR CHIRURGISCHE KLAMMERAPPLIKATOREN
MÉCANISME DE POUSSÉE POUR APPLICATEUR D'AGRAFES CHIRURGICALES

(30) Priority: 10.11.2016 TW 105136728
(43) Date of publication of application: 25.11.2020
(73) Proprietor: MEDSCOPE BIOTECH CO., LTD., Taiwan (TW)
(72) Inventor: FAN, Hong-Yang, Zhudong Township Hsinshu County 310 (TW); HUANG, Shih-Hao, Zhudong Township Hsinshu County 310 (TW)
(74) Representative: Straus, Alexander
(86) International application number: PCT/SC2017/000007
(87) International publication number: WO 2019/190363

(56) References cited:
- CN-A- 105 555 207
- US-A1- 2014 379 003
- US-A1- 2018 070 948
- US-B1- 6 277 131

## Description

### BACKGROUND OF THE INVENTION

### 1. Field of the Invention

The present invention relates to medical instruments and more particularly, to a clip pushing mechanism for surgical clip applicator.

### 2. Description of the Related Art

Conventional clip pushing mechanisms for surgical clip applicator are commonly designed to push clips from the top to the end, thus, the clips can be squeezed to shorten the pitch between each two adjacent clips. Further, these conventional clip pushing mechanisms use two different driving members, having the drawbacks of complicated structure and inaccurately pushing the clips forward, and the leading clip can be inaccurately positioned for clipping. US 6277131 B1 discloses a clip feeder arrangement for supportive receipt in a handle of a medical clip stapling gun to permit the advancement of a plurality of clips seriatim by a trigger mechanism in the handle to a location between a pair of pincher jaws. A distalmost clip of that plurality of clips is being advanced to the jaws prior to advancement of the remaining plurality of clips. The feeder arrangement comprises a ladder arranged within a U-shaped cartridge having a proximal end and a distal end. The distalmost clip is moved distally away from the ladder by a clip feeder bar mechanism which is arranged for first advancing the distalmost clip in the cartridge to a location between the jaws. The remaining plurality of clips are then also advanced distally by the ladder, both of the advancement motions occurring in a single distal advance of the clip feeder bar mechanism. US 2014379003 A1 discloses a clip applier of handle and cartridge for applying clips in surgical procedures with handle preferably a scissors type defined by a housing of shell members containing a cam operated linear translator actuated by handle levers, an anti-backup mechanism limiting handle lever movement to full pull and release strokes while preventing partial pull or release handle strokes, and with cartridge fitted with lockout mechanism to disable applier after last clip is used in surgery, with a puller bar lock to hold puller bar position in cartridge prior to assembly with handle, a detent spring mounted in cartridge cover member as part of clip handling mechanism, and a rapid-fire pawl as part of clip handling mechanism to prevent cartridge jamming when clips are applied in rapid succession.

Conventional clip pushing mechanisms for surgical clip applicator are still not satisfactory in function and have room for improvement.

### SUMMARY OF THE INVENTION

The present invention has been accomplished under the circumstances in view. It is the main object of the present invention to provide a clip pushing mechanism for surgical clip applicator, which has a simple structure, facilitates operation, and can accurately push clips to a forward step position.

To achieve this and other objects of the present invention, the present invention provides a clip pushing mechanism for surgical clip applicator for loading and pushing clips that are arranged in series with a clip pitch defined between each two adjacent clips. The clip pushing mechanism comprises a clip holder bar comprising an accommodation channel for accommodating the clip in a series, a clip feeding ladder member comprising a clip pushing portion and a plurality of driven portions spaced along the length thereof with a pitch defined between each two adjacent driven portions, and a clip pusher comprising a body, a front pusher for pushing the leading clip and a rear pusher for pushing each driven portion of the clip feeding ladder member. The clip holder bar furthermore comprises a baffle stoppable between at least one said driven portion of said clip feeding ladder member and said rear pusher of said clip pusher.

Thus, when the front pusher of the clip pusher the leading one of the series of clips, the driven portions of the clip feeding ladder member are pushed by the rear pusher of the clip pusher so that the clip pushing portion of the clip feeding ladder member accurately pushes the clips to a forward step position, achieving the objects of the present invention.

Preferably, the clip pitch is equal to the pitch between each two adjacent driven portions.

Preferably, the clip pushing mechanism further comprises a lower tube half that defines therein a sliding groove for accommodating the body of the clip pusher in an axially slidable manner.

Preferably, the clip holder bar comprises a one-way stopper for stopping against one driven portion of the clip feeding ladder member for allowing the clip feeding ladder member to be moved forward and prohibiting the clip feeding ladder member from backward displacement.

Other advantages and features of the present invention will be fully understood by reference to the following specification in conjunction with the accompanying drawings, in which like reference signs denote like components of structure.

### BRIEF DESCRIPTION OF THE DRAWINGS

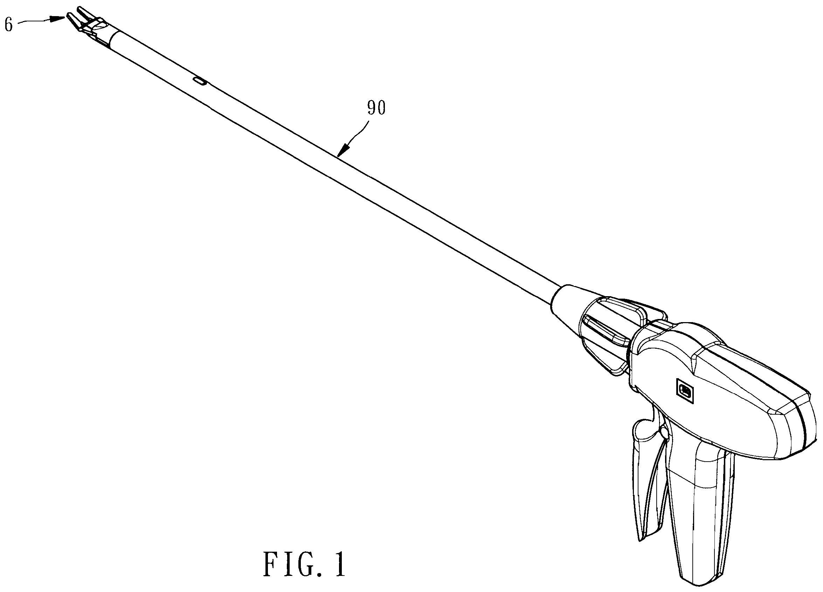

FIG 1 is an oblique top elevational view of a surgical clip applicator embodying the present invention.

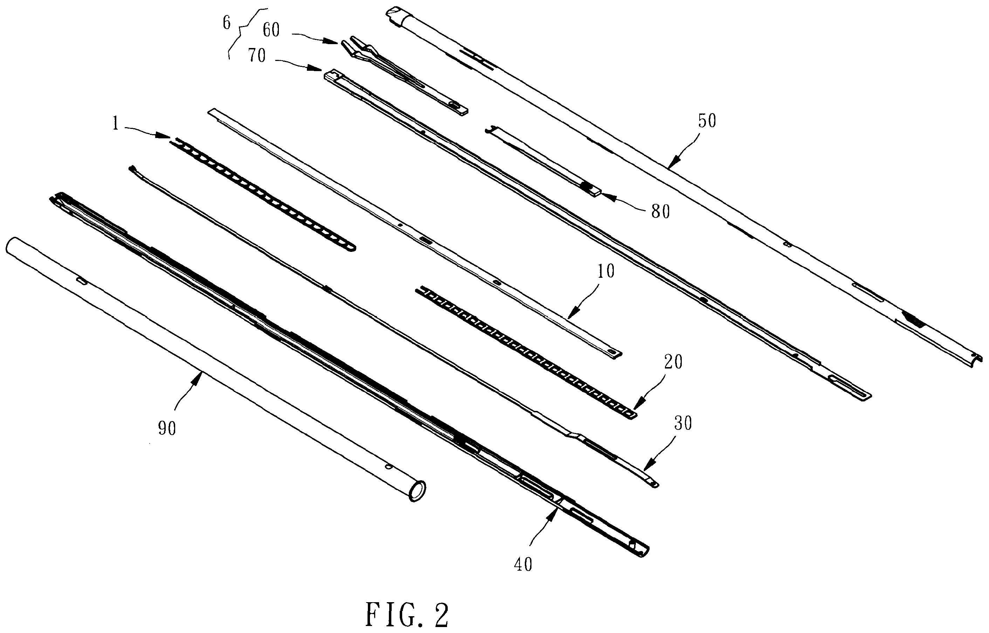

FIG. 2 is an exploded view of a clip pushing mechanism for surgical clip applicator in accordance with the present invention.

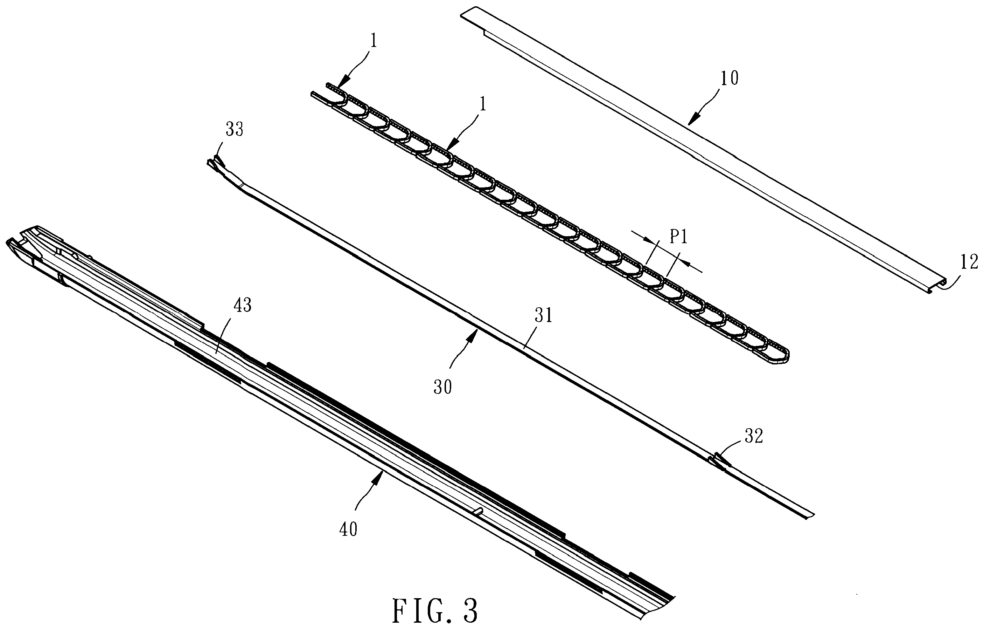

FIG 3 is an exploded view of a part of the clip pushing mechanism for surgical clip applicator in accordance with the present invention.

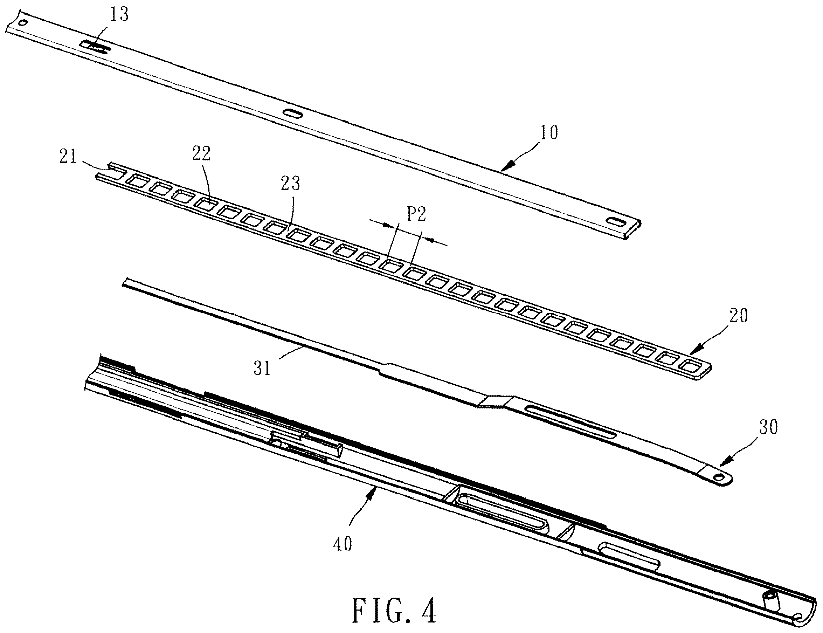

FIG. 4 is an exploded view of another part of the clip pushing mechanism for surgical clip applicator in accordance with the present invention.

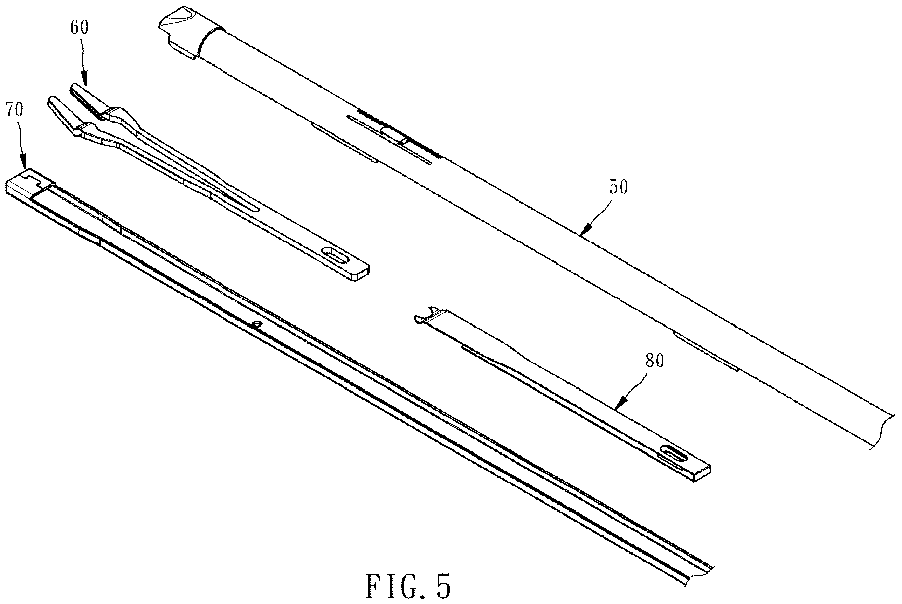

FIG 5 is an exploded view of still another part of the clip pushing mechanism for surgical clip applicator in accordance with the present invention.

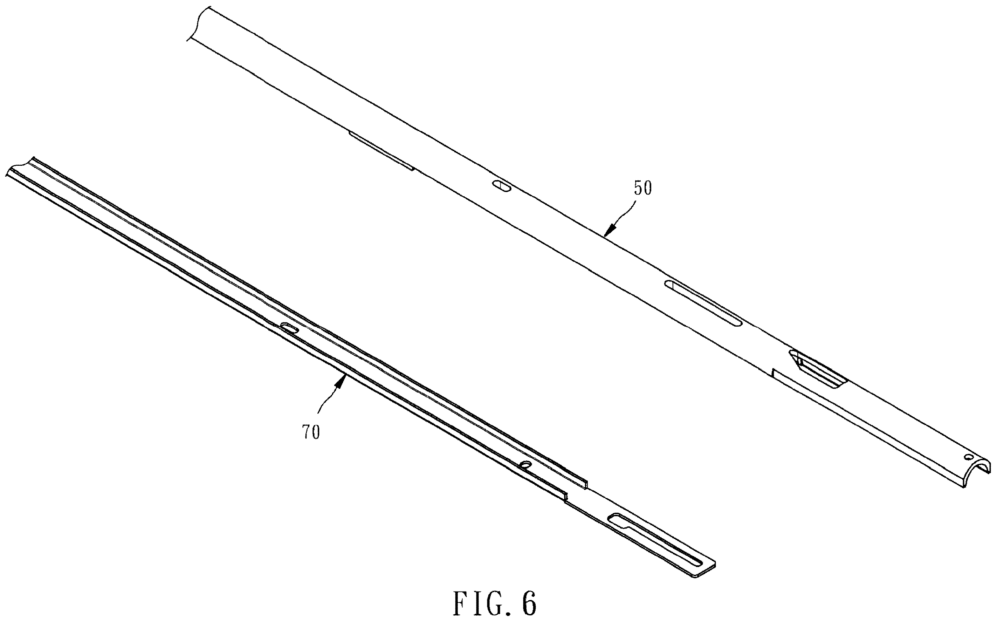

FIG 6 is an exploded view of still another part of the clip pushing mechanism for surgical clip applicator in accordance with the present invention.

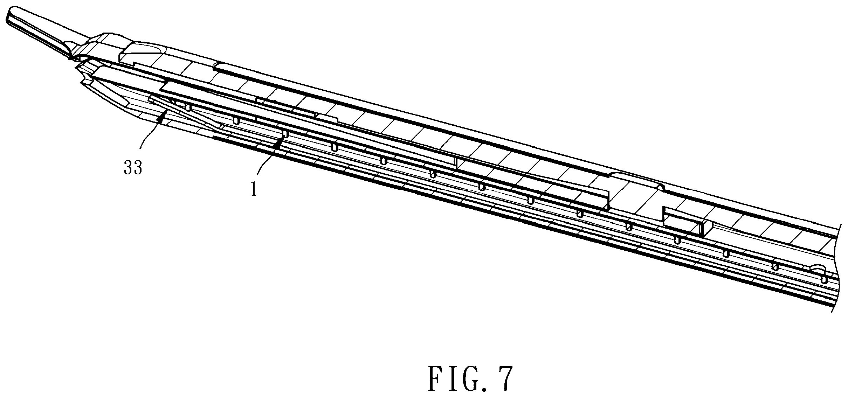

FIG. 7 is an exploded view of still another part of the clip pushing mechanism for surgical clip applicator in accordance with the present invention.

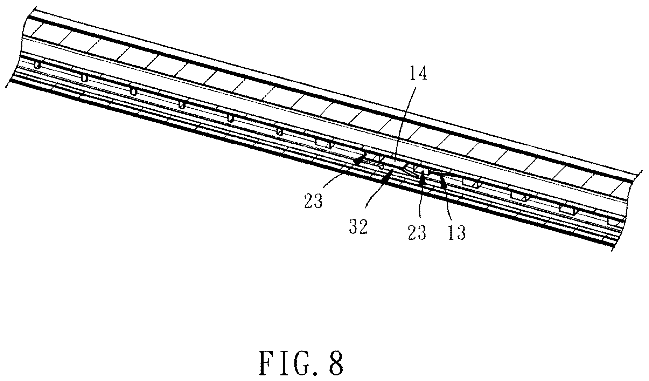

FIG. 8 is an assembly view of a part of the clip pushing mechanism for surgical clip applicator in accordance with the present invention.

### DETAILED DESCRIPTION OF THE INVENTION

Referring to the annexed drawings in detail, a clip pushing mechanism for surgical clip applicator in accordance with the present invention is adapted for loading and pushing clips 1 wherein each adjacent two clips 1 define therebetween a clip pitch P1.

The clip pushing mechanism comprises:
a clip holder bar 10 comprising accommodation channel 12 for accommodating the clips 1 in series, a one-way stopper 13 located at a middle part thereof for abutment against one driven portion 23 of a clip feeding ladder member 20 for allowing the clip feeding ladder member 20 to be moved forward and prohibiting the clip feeding ladder member 20 from backward displacement, and a baffle 14;
a clip feeding ladder member 20 comprising a clip pushing portion 21, a plurality of driven portions 23 spaced along the length thereof and stoppable by the one-way stopper 13 of the clip holder bar 10 for allowing the clip feeding ladder member 20 to be moved forward and prohibiting the clip feeding ladder member 20 from backward displacement, and a pitch P2 defined between each two adjacent driven portions 23 and equal to the clip pitch P1;
a clip pusher 30 comprising a body 31, a front pusher 33 for pushing the leading one of the loaded series of clips 1, and a rear pusher 32 for pushing each driven portion 23 of the clip feeding ladder member 20;
a lower tube half 40 comprising a sliding groove 43 for accommodating the body 31 of the clip pusher 30 in an axially slidable manner;
an upper tube half 50 mated with the lower tube half 40;
a pair of jaws 60 connected to the upper tube half 50;
a jaw bundle 70 for controlling the jaws 60 to open and close;
a clip presser 80 mounted to the upper tube half 50 and mating with the jaws 60 and the jaw bundle 70 to create a clip clamping set 6; and
an outer tube 90 attached onto the lower tube half 40 and the upper tube half 50.

Further, the baffle 14 of the clip holder bar 10 is stopped between one driven portion 23 of the clip feeding ladder member 20 and the rear pusher 32 of the clip pusher 30. The baffle 14 can stop the rear pusher 32 for at least one pitch P2 so that when the clip pusher 30 is moved through two pitches P2, it simply pushes the clip feeding ladder member 20 to move through one pitch P2.

With the above structure, the baffle 14 of the clip holder bar 10 enables the rear pusher 32 to move the clip feeding ladder member 20 through one pitch P2 when the clip pusher 30 moves through two clip pitches P1.

Thus, when the front pusher 33 of the clip pusher 30 pushes the leading clip, the driven portions 23 of the clip feeding ladder member 20 are pushed by the rear pusher 32 of the clip pusher 30, causing the clip pushing portion 21 of the clip feeding ladder member 20 accurately pushes the clips to a forward step position, achieving the objects of the present invention.

The clip pushing mechanism can be configured comprised of a part of the aforesaid component parts to achieve the same effects. In an alternate form of the present invention, a clip pushing mechanism is provided for loading and pushing clips 1 that are arranged in series with a clip pitch P1 defined between each two adjacent clips 1. The clip pushing mechanism comprises:
a clip holder bar 10 comprising accommodation channel 12 for accommodating the clips 1 in series;
a clip feeding ladder member 20 comprising a clip pushing portion 21, a plurality of driven portions 23 spaced along the length thereof, and a pitch P2 defined between each two adjacent driven portions 23 and equal to the clip pitch P1; and
a clip pusher 30 comprising a body 31, a front pusher 33 for pushing the leading one of the loaded series of clips 1, and a rear pusher 32 for pushing each driven portion 23 of the clip feeding ladder member 20.

The various embodiments of the present invention have one or multiple of the following technical features:
The clip pitch P1 is equal to the pitch P2 between each two adjacent driven portions 23.

The clip pushing mechanism further comprises a lower tube half 40 comprising a sliding groove 43 for accommodating the body 31 of the clip pusher 30 in an axially slidable manner.

The clip holder bar 10 comprises a one-way stopper 13 for abutment against one driven portion 23 of the clip feeding ladder member 20 for allowing the clip feeding ladder member 20 to be moved forward and prohibiting the clip feeding ladder member 20 from backward displacement.

## Claims

1. A clip pushing mechanism for use in a surgical clip applicator for loading and pushing clips (1) that are arranged in series with a clip pitch (P1) defined between each two adjacent said clips (1), the clip pushing mechanism comprising:
a clip holder bar (10) comprising an accommodation channel (12) for accommodating said clips (1) in a series;
a clip feeding ladder member (20) comprising a clip pushing portion (21) and a plurality of driven portions (23) spaced along a length thereof with a pitch (P2) defined between each two adjacent said driven portions (23); and
a clip pusher (30) comprising a body (31), a front pusher (33) for pushing a leading clip of said clips (1), and a rear pusher (32) for pushing each said driven portion (23) of said clip feeding ladder member (20);
**characterized in that** said clip holder bar (10) comprises a baffle (14) stoppable between said rear pusher (32) of said clip pusher (30) and at least one of said driven portions (23) of said clip feeding ladder member (20).

2. The clip pushing mechanism as claimed in claim 1, **characterized in that** said clip feeding ladder member (20) comprises a plurality of openings (22); each said driven portion (23) is disposed between two said openings (22); said clip pusher (30) is abutted against one said driven portion (22) of said clip feeding ladder member (20).

3. The clip pushing mechanism as claimed in claim 2, **characterized in that** said clip pitch (P1) is equal to the pitch (P2) between each two adjacent said driven portions (23).

4. The clip pushing mechanism as claimed in claim 1, **characterized in that** said clip pitch (P1) is equal to the pitch (P2) between each two adjacent said driven portions (23).

5. The clip pushing mechanism as claimed in claim 1, 2, 3 or 4, **characterized by** further comprising a lower tube half (40) defining therein a sliding groove (43) for accommodating said body (31) of said clip pusher (30) in an axially slidable manner.

6. The clip pushing mechanism as claimed in claim 5, **characterized in that** said clip holder bar (10) comprises a one-way stopper (13) for stopping against one said driven portion (23) of said clip feeding ladder member (20) for allowing said clip feeding ladder member (20) to be moved forward and prohibiting said clip feeding ladder member (20) from backward displacement.

7. The clip pushing mechanism as claimed in claim 1, 2, 3 or 4, **characterized in that** said clip holder bar (10) comprises a one-way stopper (13) for stopping against one said driven portion (23) of said clip feeding ladder member (20) for allowing said clip feeding ladder member (20) to be moved forward and prohibiting said clip feeding ladder member (20) from backward displacement.

## Patentansprüche

1. Clip-Schiebemechanismus zur Verwendung in einem chirurgischen Clip-Applikator zum Laden und Schieben von Clips (1), die in einer Reihe mit einem zwischen jeweils zwei benachbarten Clips (1) definierten Clip-Abstand (P1) angeordnet sind, worin der Clip-Schiebemechanismus umfasst:
eine Clip-Halteleiste (10) mit einem Aufnahmekanal (12) zum Aufnehmen der Clips (1) in einer Reihe;
ein Clip-Zuführungsleiterelement (20) mit einem Clip-Schubabschnitt (21) und mehreren angetriebenen Abschnitten (23), die entlang seiner Länge mit einem Abstand (P2) zwischen jeweils zwei benachbarten angetriebenen Abschnitten (23) angeordnet sind; und
einen Clip-Schieber (30) mit einem Körper (31), einem vorderen Schieber (33) zum Schieben eines vorderen Clips der Clips (1) und einem hinteren Schieber (32) zum Schieben jedes angetriebenen Abschnitts (23) des Clip-Zuführungsleiterelements (20);
**dadurch gekennzeichnet, dass** die Clip-Halteleiste (10) eine Ablenkplatte (14) umfasst, die zwischen dem hinteren Schieber (32) des Clip-Schiebers (30) und mindestens einem angetriebenen Abschnitt (23) des Clip-Zuführungsleiterelements (20) angehalten werden kann.

2. Clip-Schiebemechanismus nach Anspruch 1, **dadurch gekennzeichnet, dass** das Clip-Zuführleiterelement (20) mehrere Öffnungen (22) umfasst; jeder angetriebene Abschnitt (23) zwischen zwei der Öffnungen (22) angeordnet ist; der Clip-Schieber (30) an einem der angetriebenen Abschnitte (22) des Clip-Zuführleiterelements (20) anliegt.

3. Clip-Schiebemechanismus nach Anspruch 2, **dadurch gekennzeichnet, dass** der Clip-Abstand (P1) gleich dem Abstand (P2) zwischen jeweils zwei benachbarten angetriebenen Abschnitten (23) ist.

4. Clip-Schiebemechanismus nach Anspruch 1, **dadurch gekennzeichnet, dass** der Clip-Abstand (P1) gleich dem Abstand (P2) zwischen jeweils zwei benachbarten angetriebenen Abschnitten (23) ist.

5. Clip-Schiebemechanismus nach Anspruch 1, 2, 3 oder 4, **dadurch gekennzeichnet, dass** dieser ferner eine untere Rohrhälfte (40) umfasst, in der eine Gleitrille (43) ausgebildet ist, um den Körper (31) des Clip-Schiebers (30) axial verschiebbar aufzunehmen.

6. Clip-Schiebemechanismus nach Anspruch 5, **dadurch gekennzeichnet, dass** die Clip-Halteleiste (10) einen Einwegstopper (13) umfasst, der zum Stoppen gegen einen der angetriebenen Abschnitte (23) des Clip-Zuführungsleiterelements (20) dient, damit das Clip-Zuführungsleiterelement (20) vorwärtsbewegt werden kann und eine Rückwärtsbewegung des Clip-Zuführungsleiterelements (20) verhindert wird.

7. Clip-Schiebemechanismus nach Anspruch 1, 2, 3 oder 4, **dadurch gekennzeichnet, dass** die Clip-Halteleiste (10) einen Einwegstopper (13) umfasst, der zum Stoppen gegen einen der angetriebenen Abschnitte (23) des Clip-Zuführungsleiterelements (20) dient, damit das Clip-Zuführungsleiterelement (20) vorwärtsbewegt werden kann und eine Rückwärtsbewegung des Clip-Zuführungsleiterelements (20) verhindert wird.

## Revendications

1. Mécanisme de poussée de clips destiné à être utilisé dans un applicateur chirurgical de clips pour charger et pousser des clips (1) qui sont disposés en série avec un pas de clip (P1) défini entre chacun desdits clips (1) adjacents, le mécanisme de poussée de clips comprenant:
une barre de support de clips (10) comprenant un canal de logement (12) pour loger lesdits clips (1) en série;
un élément d'échelle d'alimentation en clips (20) comprenant une partie de poussée de clip (21) et une pluralité de parties entraînées (23) espacées sur sa longueur avec un pas (P2) défini entre chacune desdites parties entraînées adjacentes (23); et
un poussoir de clips (30) comprenant un corps (31), un poussoir avant (33) pour pousser un clip de tête desdits clips (1), et un poussoir arrière (32) pour pousser chacune desdites parties entraînées (23) dudit élément d'échelle d'alimentation en clips (20);
**caractérisé en ce que** ladite barre de support de clips (10) comprend un déflecteur (14) pouvant être arrêté entre ledit poussoir arrière (32) dudit poussoir de clips (30) et au moins une dite partie entraînée (23) dudit élément d'échelle d'alimentation en clips (20).

2. Mécanisme de poussée de clips selon la revendication 1, **caractérisé en ce que** ledit élément d'échelle d'alimentation en clips (20) comprend une pluralité d'ouvertures (22); chacune desdites parties entraînées (23) est disposée entre deux desdites ouvertures (22); ledit poussoir de clips (30) est en butée contre une dite partie entraînée (22) dudit élément d'échelle d'alimentation en clips (20).

3. Mécanisme de poussée de clips selon la revendication 2, **caractérisé en ce que** ledit pas de clip (P1) est égal au pas (P2) entre chacune des deux parties entraînées adjacentes (23).

4. Mécanisme de poussée de clips selon la revendication 1, **caractérisé en ce que** ledit pas de clip (P1) est égal au pas (P2) entre chacune des deux parties entraînées adjacentes (23).

5. Mécanisme de poussée de clips selon la revendication 1, 2, 3 ou 4, **caractérisé en ce qu'**il comprend en outre une moitié de tube inférieure (40) définissant à l'intérieur une rainure coulissante (43) pour recevoir ledit corps (31) dudit poussoir de clips (30) de manière à pouvoir coulisser axialement.

6. Mécanisme de poussée de clips selon la revendication 5, **caractérisé en ce que** ladite barre de support de clips (10) comprend un arrêt unidirectionnel (13) destiné à s'arrêter contre l'une desdites parties entraînées (23) dudit élément d'échelle d'alimentation en clips (20) afin de permettre audit élément d'échelle d'alimentation en clips (20) d'être déplacé vers l'avant et d'empêcher ledit élément d'échelle d'alimentation en clips (20) de se déplacer vers l'arrière.

7. Mécanisme de poussée de clips selon les revendications 1, 2, 3 ou 4, **caractérisé en ce que** ladite barre de support de clips (10) comprend un arrêt unidirectionnel (13) destiné à s'arrêter contre l'une desdites parties entraînées (23) dudit élément d'échelle d'alimentation en clips (20) afin de permettre audit élément d'échelle d'alimentation en clips (20) d'être déplacé vers l'avant et d'empêcher ledit élément d'échelle d'alimentation en clips (20) de se déplacer vers l'arrière.
